# EUROPEAN PATENT APPLICATION

(11) **EP 4 331 628 A1**
(43) Date of publication of application: **06.03.2024**
(21) Application number: 22795264.5
(22) Date of filing: 28.02.2022
(51) Int. Cl.: A61L 2/10, A61L 2/18, A61L 9/20

(54) **INACTIVATION METHOD**

(30) Priority: 30.04.2021 JP 2021077178
(71) Applicant: Ushio Denki Kabushiki Kaisha, Tokyo 100-8150 (JP)
(72) Inventor: OHASHI, Hiroyuki, Tokyo 100-8150 (JP); SUZUKI, Shinji, Tokyo 100-8150 (JP); ABE, Ryoji, Tokyo 100-8150 (JP); OWADA, Tatsushi, Tokyo 100-8150 (JP)
(74) Representative: Maiwald GmbH
(86) International application number: PCT/JP2022/008139
(87) International publication number: WO 2022/230341

(57) **Abstract**

There is provided an inactivation method that is capable of inactivating and sterilizing microorganisms and/or viruses existing in the space where patients are transported and/or treated in a short period of time and safely to humans. The inactivation method inactivates harmful microorganisms and/or viruses existing in an enclosed space where a patient is transported and/or treated, and comprises: a first step of performing a wiping operation that wipes down a surface in the enclosed space with a sterilizing solution after transporting and/or treating the patient; a second step of irradiating, simultaneously with the first step, droplets that scatter from a wiping area of the surface during the wiping operation in the first step with ultraviolet light in a wavelength range between 190 nm and 235 nm as ultraviolet light having a wavelength that inactivates harmful microorganisms and/or viruses.

## Description

### TECHNICAL FIELD

The present invention relates to an inactivation method for inactivating harmful microorganisms and/or viruses.

### BACKGROUND ART

Microorganisms (e.g., bacteria, fungi, and the like) and/or viruses present in a space or on an object surface may cause infectious diseases to humans or non-human animals, and concerns may arise that the spread of infectious diseases would threaten human lives. In particular, droplets (including sputum, nasal mucus, vomit, and the like) that contain harmful microorganisms and/or viruses may be dispersed from patients and remain in or on surfaces of enclosed spaces where patients with infectious diseases have been transported or treated. Such remaining harmful droplets are likely to cause secondary infection to the next person who enters the enclosed space.

For example, conventionally in ambulances, a sterilization operation is performed by wiping down a whole interior of the vehicle with an ethanol or chlorine disinfectant after a patient has been transported. Such sterilization operation is assumed to be mainly performed by emergency service workers. However, since there are various pieces of equipment (e.g., stretchers, chairs, handrails, drawers, measuring instruments, and the like) inside the ambulance, wiping down those surfaces would be a complicated and labor-intensive task. In addition, such sterilization operation involves the risk of infection, which makes the sterilization workers feel unsafe.

On the other hand, a certain technology has been known that inactivates harmful microorganisms (e.g., bacteria, mold, and the like) and/or viruses floating in a space or adhered to surfaces such as floors and walls by irradiating them with ultraviolet light.

Patent Literature 1 (US Patent No. 9855353 B) discloses a sterilization system in which ultraviolet-C (i.e., UV-C) lamps are installed on a ceiling inside an ambulance and UV-C ultraviolet light is irradiated inside the unmanned ambulance to sterilize surfaces inside the ambulance.

### LISTING OF REFERENCES

### PATENT LITERATURE

PATENT LITERATURE 1: US Patent No. 9855353 B

### SUMMARY OF THE INVENTION

### PROBLEMS TO BE SOLVED BY THE INVENTION

For example, when the frequency of emergency calls increases, ambulances need to transport the next patient immediately after the patient has been transported, and the sterilization operation inside the vehicle needs to be performed in a short period of time.

However, performing the sterilization operation by wiping in a short period of time would increase the workload on sterilization workers. In addition, according to the technology disclosed in the above Patent Literature 1, the sterilization operation cannot be performed unless the vehicle is in an unmanned state where no human is present, which means that the ambulance cannot be dispatched until the sterilization operation is completed, making it impossible to operationalize the ambulance efficiently.

The present invention has been made in order to solve the above mentioned problems and an object thereof is to provide an inactivation method that is capable of inactivating microorganisms and/or viruses existing in the space where patients are transported and/or treated and sterilizing the space in a short period of time and safely to humans.

### SOLUTION TO PROBLEMS

In order to solve the above mentioned problems, according to one aspect of the present invention, there is provided an inactivation method of inactivating harmful microorganisms and/or viruses existing in an enclosed space where a patient is transported and/or treated, comprising: a first step of performing a wiping operation that wipes down a surface in the enclosed space with a sterilizing solution after transporting and/or treating the patient; a second step of irradiating, simultaneously with the first step, droplets that scatter from wiping an area of the surface during the wiping operation in the first step with ultraviolet light in a wavelength range between 190 nm and 235 nm as ultraviolet light having a wavelength that inactivates harmful microorganisms and/or viruses.

In this way, by simultaneously performing the wiping operation using the sterilizing solution and the sterilization and inactivation processing using ultraviolet light, it makes it possible to appropriately perform the sterilization operation of enclosed spaces where patients are transported and/or treated in a short period of time. Furthermore, since the ultraviolet light having the wavelength range between 190 nm and 235 nm is emitted, which have little adverse effects on cells of humans and animals, it makes it possible to perform the sterilization and inactivation safely to the workers who perform the wiping operation.

Yet furthermore, objects to be cleaned that exist in the enclosed space where patients are transported and/or treated are likely to contain droplets ejected from patients' coughs, sneezes, and conversations, and in some cases, such droplets may contain harmful microorganisms and/or viruses. By irradiating the droplets scattered from the wiping area with ultraviolet light during the wiping operation, it makes it possible to inactivate the above microorganisms and/or viruses by the ultraviolet light before the above microorganisms and/or viruses are inhaled by the workers, even when the droplets contain harmful microorganisms and/or viruses. As a result, it makes it possible to appropriately prevent secondary infection to workers.

Furthermore, the above inactivation method may further comprise: a third step of irradiating, prior to the first step and the second step, the enclosed space with the ultraviolet light in an unmanned state where no human is present in the enclosed space after transporting and/or treating the patient.

In this case, the worker who is in charge of performing the wiping operation is able to enter the enclosed space that has been irradiated with the ultraviolet light and decontaminated to some extent to perform the wiping operation. Therefore, it makes it possible to reduce the risk of infection for the workers and also to alleviate the psychological anxiety of the workers.

Yet furthermore, in the above inactivation method, the enclosed space may be a space where a shelf storing equipment used for a treatment for the patient is arranged, the inactivation method may further comprise a fourth step of exposing an interior of the shelf after transporting and/or treating the patient, and the third step may irradiate an area including the exposed interior of the shelf with the ultraviolet light.

In this case, it makes it possible to appropriately inactivate microorganisms and/or viruses existing in the interior spaces and surfaces of shelves (including drawers, and the like). Therefore, it makes it possible to eliminate or at least simplify the wiping operation of the interior of shelves.

Yet furthermore, in the above inactivation method, the sterilizing solution may be a volatile sterilizing solution.

In this case, it makes it possible to irradiate droplets that spread out when the sterilizing solution sprayed on the wiping area volatilizes during the wiping operation with the ultraviolet light.

Yet furthermore, in the above inactivation method, the second step may irradiate the droplets with the ultraviolet light by irradiating a space between the wiping area and a worker performing the wiping operation.

In this case, it makes it possible to inactivate microorganisms and/or viruses contained in the droplets by irradiating with ultraviolet light more appropriately before the worker inhales the droplets that scatter from the wiping area during the wiping operation.

Yet furthermore, in the above inactivation method, the first step may perform the wiping operation selectively on an area of the surface in the enclosed space where inactivation of the microorganisms and/or viruses by irradiation with the ultraviolet light is determined to be insufficient.

In this case, it makes it possible to reduce the burden of the wiping operation for the workers.

Yet furthermore, in the above inactivation method, the enclosed space may be a space inside an ambulance.

In this case, it makes it possible to appropriately perform the sterilization operation inside the vehicle after transporting a patient in a short period of time to allow the next patient to be transported in a timely manner. Thus, it makes it possible to operationalize ambulances more efficiently.

### ADVANTAGEIOUS EFFECT OF THE INVENTION

According to the present invention, it makes it possible to inactivate microorganisms and/or viruses existing in the space where patients are transported and/or treated and sterilize the space in a short period of time and safely to humans.

### BRIEF DESCRIPTION OF DRAWINGS

FIG. 1 is a schematic diagram illustrating an exemplary interior of an ambulance in which the ultraviolet light irradiation apparatus according to the present embodiment is installed.
FIG. 2 is a schematic diagram illustrating an exemplary exterior of the ultraviolet light irradiation apparatus according to the present embodiment.
FIG. 3 is a schematic diagram exemplarily illustrating a process of irradiating an enclosed space in an unmanned state with ultraviolet light.
FIG. 4 is a schematic diagram exemplarily illustrating processes of simultaneously performing the ultraviolet light irradiation and a wiping operation.

### DESCRIPTION OF EMBODIMENTS

Hereinafter, non-limiting embodiments of the present invention will be described in detail with reference to the accompanying drawings.

The present embodiment will exemplarily describe an inactivation method that inactivates harmful microorganisms and/or viruses existing in an ambulance, which is an enclosed space where patients are transported and treated.

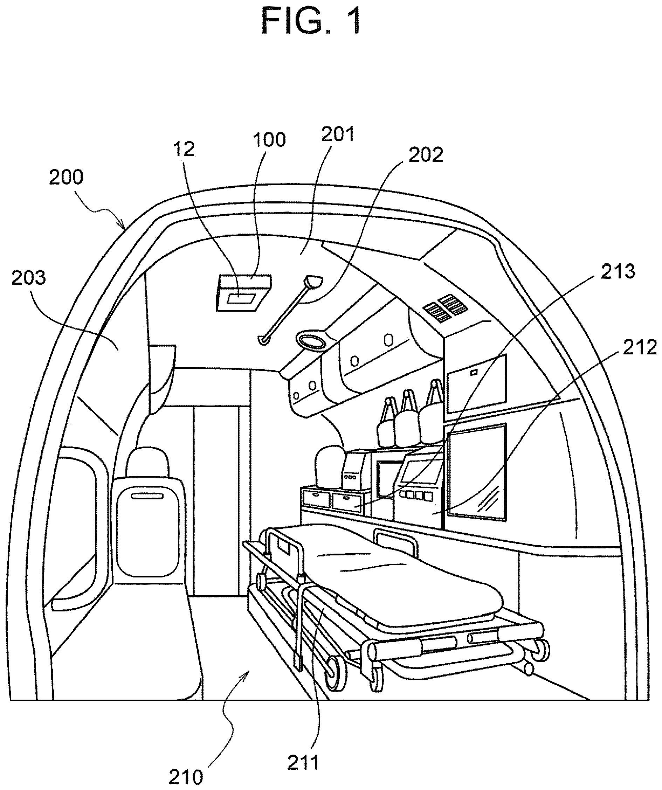

FIG. 1 is a schematic diagram illustrating an exemplary interior of an ambulance in which the ultraviolet light irradiation apparatus according to the present embodiment is installed.

The ultraviolet light (hereinafter referred to as "UV light" or simply referred to as "UV") irradiation apparatus 100 is an apparatus that performs the UV irradiation in a space where a human or an animal is present (according to the present embodiment, e.g., in an ambulance) to inactivate harmful microorganisms and/or viruses that exist in the space or on surfaces of objects in the space concerned. Here, the above objects include human bodies, animals, goods, and any other objects.

The UV irradiation apparatus 100 irradiates UV light having a wavelength between 190 nm and 235 nm (more preferably, UV light having the wavelength between 200 nm and 230 nm), which has little adverse effects on cells of humans and animals, into a target space from a light emitting surface 12.

The term "inactivation" according to the present embodiment refers to the death of microorganisms and/or viruses (or the loss of infectivity and/or toxicity thereof).

Referring to FIG. 1, the UV irradiation apparatus 100 is installed, for example, on a ceiling 201 inside an ambulance 200 and emits UV light downwardly. The UV light emitted from the UV irradiation apparatus 100 is irradiated onto surfaces (e.g., floors, walls, equipment surfaces, or the like) and spaces inside the ambulance 200.

It should be noted that the location where the UV irradiation apparatus 100 is to be installed is not limited to the ceiling 201 but may be installed in any desired location. For example, when the UV irradiation apparatus 100 is to be installed in an upper position near the ceiling 201, the UV irradiation apparatus 100 may be arranged on a handrail (or pole) 202 attached to the ceiling 201 or on the upper side of a wall 203.

At the rear of the ambulance 200 is a treatment chamber 210 on which a stretcher (or gurney) 211, which is equipment used to transport patients, may be placed. The treatment chamber 210 is equipped with various equipment 212 used to treat patients, such as cardiopulmonary resuscitators and ventilators, or shelves (e.g., drawers) 213 for storing medical equipment including gauze and bandages to protect wounds, or the like.

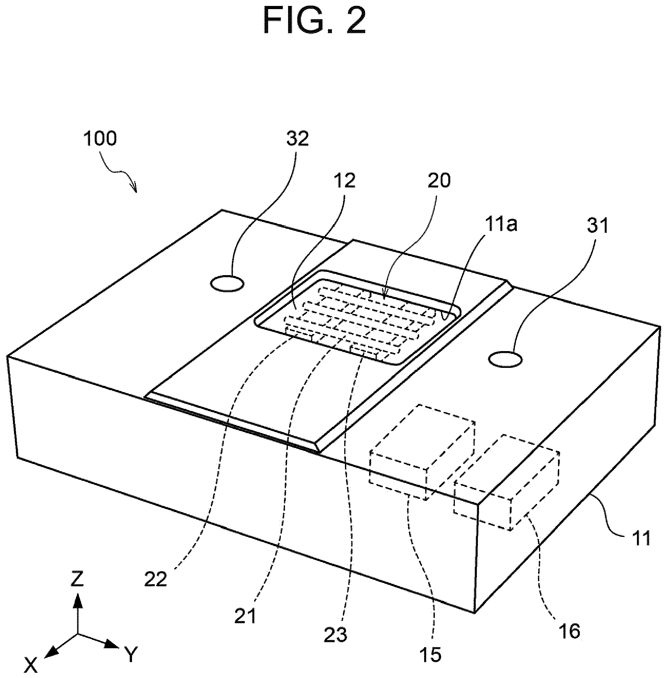

FIG. 2 is a schematic diagram illustrating an exemplary exterior of the UV irradiation apparatus 100 according to the present embodiment.

As shown in FIG. 2, the UV irradiation apparatus 100 includes a housing 11. The housing 11 is provided with an aperture 11a, and the aperture 11a is provided with a light emitting surface 12 that emits UV light. The light emitting surface 12 is constituted with a window member made of, for example, quartz glass. Also, the light emitting surface 12 may be provided with an optical filter or the like to block light having an undesired wavelength band.

An excimer lamp 20 is housed inside the housing 11 as the UV light source. The excimer lamp 20 may be, for example, a KrCI excimer lamp that emits UV light having a center wavelength of 222 nm. It should be noted that the UV light source is not limited to the KrCI excimer lamp but may be any light source that emits UV light in the wavelength range between 190 nm and 235 nm.

It should be noted that the housing 11 and the UV light source (e.g., excimer lamp 20) may constitute the light source unit.

The power of UV light to penetrate into cells differs depending on the wavelength, with shorter wavelengths having less penetrating power. For example, short wavelength UV light of approximately 200 nm, while passing through water very efficiently, is largely absorbed by the outer parts of human cells (i.e., cell cytoplasm) and thus may not have enough energy to reach the cell nuclei, which contain UV sensitive DNAs. For this reason, the above short wavelength UV light has little adverse effects on human cells. On the other hand, the UV light having the wavelength exceeding 240 nm is hardly absorbed by the outer parts of human cells (i.e., cell cytoplasm) and thus considerably penetrates into human cells. Therefore, the UV light having the wavelength exceeding 240 nm reaches the inside of human cells, and thus DNAs in cell nuclei of humans are likely to be damaged.

On the other hand, in the presence of UV light having the wavelength less than 190 nm, oxygen molecules contained in the atmosphere are photolyzed to produce more oxygen atoms, and the bonding reaction between oxygen molecules and oxygen atoms results in producing more ozone (O₃) inevitably. For this reason, it is undesirable to emit the UV light having the wavelength less than 190 nm in the atmosphere. Therefore, the UV light having the wavelength between 190 nm and 240 nm is assumed to be safe wavelength range for the humans and animals.

According to the present embodiment, as the UV light source, a UV light source that emits the UV light having a peak wavelength in the wavelength range between 190 nm and 235 nm is used, which has less adverse effects on the human bodies and provides an inactivation effect. Furthermore, it is preferable to use UV light having a peak wavelength of 200 nm or longer to more effectively suppress ozone generation in the atmosphere. Yet furthermore, it is preferable to use UV light having a shorter wavelength band as a wavelength band with higher safety. For example, a UV light source having a peak wavelength in the wavelength range between 200 nm and 230 nm may be used.

The excimer lamp 20 is equipped with a discharge vessel 21, which is a straight circular tube airtightly sealed at both ends. The discharge vessel 21 may be made of, for example, quartz glass. The inside of the discharge vessel 21 is filled with a rare gas and halogen as the luminous gas. According to the present embodiment, krypton chloride (KrCI) gas is used as the luminous gas. In this case, the peak wavelength of emitted light obtainable from the luminous gas is 222 nm.

It should be noted that the luminous gas is not limited to the above. For example, krypton bromide (KrBr) gas may be used as the luminous gas. In the case of KrBr excimer lamps, the peak wavelength of emitted light obtainable from the luminous gas is 207 nm.

Although the UV irradiation apparatus 100 is illustrated to be equipped with multiple (i.e., three) discharge vessels 21 in FIG. 2, the number of discharge vessels 21 is not limited thereto.

A pair of electrodes (i.e., first electrode 22 and second electrode 23) are arranged in contact with the outer surface of the discharge vessel 21. The first electrode 22 and the second electrode 23 are arranged on the side opposite the light extraction surface in the discharge vessel 21 (i.e., the side in the -Z direction), spaced from each other in the tube axis direction (i.e., Y direction) of the discharge vessel 21.

Furthermore, the discharge vessel 21 is arranged to straddle those two electrodes 22 and 23 while contacting the two electrodes 22 and 23. More specifically, each of the two electrodes 14B and 15B has a concave groove and the discharge vessel 21 is fitted into the concave grooves of the electrodes 22 and 23.

Out of the pair of electrodes, one electrode (e.g., first electrode 22) is the high voltage side electrode and the other electrode (e.g., second electrode 23) is the low voltage side electrode (i.e., ground electrode). Applying a high frequency voltage between the first electrode 22 and the second electrode 23, the excimer lamp is lit.

The light extraction surface of the excimer lamp 20 is positioned opposite the light emitting surface 12. Therefore, the light emitted from the excimer lamp 20 is emitted from the UV irradiation apparatus 100 through the light emitting surface 12.

Here, the first and second electrodes 22 and 23 may be made of metal members that are reflective to the light emitted from the excimer lamp 21, respectively. In this case, the light emitted from the discharge vessel 21 in the -Z direction can be reflected and travel in the +Z direction.

Furthermore, the light emitting surface 12 may be provided with an optical filter as described above. The optical filter may be, for example, a wavelength selective filter that transmits light having the wavelength range between 190 nm and 235 nm (more preferably, light having the wavelength range between 200 nm and 230 nm), which has less adverse effects on the human bodies, and cuts the UV-C wavelength range between 236 nm and 280 nm. More specifically, the UV irradiance of respective wavelengths of 236 nm to 280 nm is reduced to 1% or less with respect to the UV irradiance of the peak wavelengths in the wavelength range between 190 nm and 235 nm.

As the wavelength selective filter, for example, an optical filter with a dielectric multilayer film made of HfO₂ layers and SiO₂ layers may be used.

It should be noted that, alternatively, an optical filter with a dielectric multilayer film made of SiO₂ layers and Al₂O₃ layers may be used as the wavelength selective filter.

In this way, by providing the optical filter on the light emitting surface 12 of the UV irradiation apparatus 100, it makes it possible to suppress the light from leaking out of the housing 11 more reliably even when light containing harmful components to humans is emitted from the excimer lamp 20.

The UV irradiation apparatus 100 is also equipped with a power supply unit 15 and a control unit 16, as shown in FIG. 2.

The power supply unit 15 includes a power component such as an inverter to which power is supplied from a power source and a cooling component such as a heat sink to cool the power component. The control unit 16 controls the lighting of the excimer lamp 20 which constitutes the light source unit.

Here, the control unit 16 may perform so-called continuous lighting, in which light emission from the light emitting surface 12 is continuous, or intermittent lighting, in which light emission from the light emitting surface 12 is intermittent, alternating between light-emitting and non-emitting operations.

Furthermore, the UV irradiation apparatus 100 may be equipped with a human-detecting sensor 31 and a proximity sensor 32. The human-detecting sensor 31 and the proximity sensor 32 may be arranged, for example, in the vicinity of the light emitting surface 12 of the housing 11.

The human-detecting sensor 31 detects a human present in a certain area (i.e., irradiation area) where the UV light emitted from the light emitting surface 12 is irradiated. The human-detecting sensor 31 may be, for example, a pyroelectric infrared sensor that detects changes in heat (i.e., infrared rays) radiated from the human bodies or the like. When the human-detecting sensor 31 detects the presence of a human, the human-detecting sensor 31 transmits a detection signal to the control unit 16.

The proximity sensor 32 detects objects in the detection range, which is the area within a predetermined separation distance from the light emitting surface 12. Here, the objects include humans, animals, and any other objects. The above predetermined separation distance may be set as appropriate according to the installation environment of the UV irradiation apparatus 100 (e.g., the size of the installation space), the irradiance of UV light on the light emitting surface 12, and the like. When the UV irradiation apparatus 100 is installed in a space with a low ceiling, such as in the ambulance 200, and the UV light is irradiated at a relatively high irradiance, the above predetermined separation distance may be, for example, a proximity distance of approximately 5 cm to 50 cm.

The proximity sensor 32 is able to detect the separation distance from the light emitting surface 12 to the target object in a direction orthogonal to the light emitting surface 12. The above predetermined separation distance is set to be closer to the light emitting surface 12 than the distance from which the UV light emitted from the light emitting surface 12 is reachable. In other words, the detection range of the proximity sensor 32 is set within the irradiation area where the UV light emitted from the light emitting surface 12 is irradiated.

The proximity sensor 32 may be, for example, an infrared proximity sensor that includes an infrared light emitting element such as an infrared LED and a light receiving element such as a photodiode and detects the distance to the target object by receiving the infrared light emitted from the infrared light emitting element and reflected by the target object with the light receiving element. When the proximity sensor 32 detects an object within the detection range, the proximity sensor 32 transmits a detection signal to the control unit 16.

It should be noted that the proximity sensor 32 is not limited to the infrared type described above, as long as the proximity sensor 32 is able to detect whether or not the target object is present in the detection range. The proximity sensor 32 may be an ultrasonic type or a radio wave type, for example.

In order to prevent secondary infection, the interior of an ambulance is required to be inactivated to inactivate harmful microorganisms and/or viruses in the space and on surfaces after the patient has been transported and before the next patient is transported.

A certain technology has been known that sterilizes surfaces inside an unmanned ambulance by installing a UV-C lamp on the ceiling of the ambulance and irradiating the surfaces inside the unmanned ambulance with the UV-C ultraviolet light. However, due to the complexity of the interior of the ambulance with various equipment (e.g., stretchers, chairs, handrails, drawers, measuring instruments, and the like), there may be areas that are not sufficiently exposed to the UV light, such as areas that are shaded from the light source or inside drawers. In addition, there may be large droplets (e.g., sputum, nasal mucus, vomit, or the like) in the ambulance that cannot be sterilized or inactivated by irradiation with the UV light alone. Therefore, decontamination by irradiation with the UV light alone is considered to be insufficient, and it is necessary to wipe the interior of the vehicle with ethanol or chlorine disinfectant.

Such wiping operation, however, is assumed to be performed by certain workers such as emergency service workers, and is both time-consuming and labor-intensive. When the wiping operation takes a long time after a patient has been transported to the hospital, the next patient cannot be transported in a timely manner and the ambulance cannot be operationalized efficiently. In addition, manual wiping operations in a vehicle that has not yet been decontaminated entail a higher risk of infection, causing psychological anxiety among the workers.

Therefore, according to the present embodiment, after a patient is transported to the hospital or the like, UV irradiation is first performed inside the ambulance 200 in an unmanned state, and after the decontamination processing has been completed to a certain extent, workers are to enter the interior of the ambulance 200 to perform the wiping operation. At this time, the wiping operation and the UV irradiation are performed simultaneously.

More specifically, after the patient has been transported to the hospital or other destination and removed from the ambulance, the control unit 16 of the UV irradiation apparatus 100 turns on the excimer lamp 20 to irradiate the interior of the unmanned vehicle with the UV light.
Here, the control unit 16 may turn on the excimer lamp 20 based on a lighting instruction (e.g., by operating a lighting switch) from an emergency service worker who is the last to exit the vehicle, or alternatively, the control unit 16 may turn on the excimer lamp 20 when it is detected by various sensors that the patient has been removed and the vehicle is unmanned.

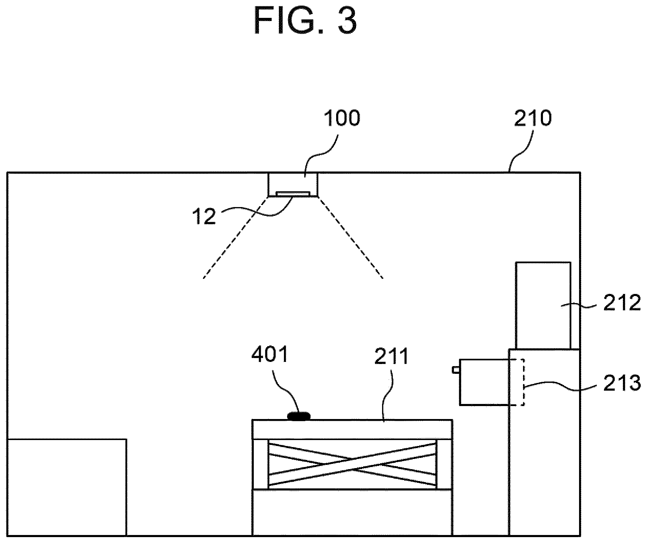

This UV irradiation inactivates bacteria and viruses adhering to the surfaces of various equipment located in the vehicle or floating in the space therein. In this case, however, the UV light is not irradiated onto the interior of the drawers in the treatment chamber 210. Therefore, after removing the patient, the emergency service worker may open the drawer 213 in treatment chamber 210 and expose the interior of the drawer 213 before exiting the treatment chamber 210, as shown in FIG. 3. This allows the exposed interior of the drawer 213 to be irradiated with the UV light.

Subsequently, after a certain period of time of the UV irradiation in an unmanned state, the worker who performs the wiping operation enters the treatment chamber 210. Here, the above certain period of time for the UV irradiation in an unmanned state is set to the time necessary to inactivate microorganisms and/or viruses in the space to the extent, which is considered to raise no problem even if a human enters (e.g., 90% sterilization).

In this case, for example, the worker himself/herself may count the above certain period of time and then enter the treatment chamber 210, or alternatively, the UV irradiation apparatus 100 may count the UV irradiation time and signal the worker that he/she may enter when the above certain period of time elapses. The signal to the worker may be, for example, by lighting a notification indicator lamp installed at an appropriate location that is easily visible to the worker, changing the color of the above indicator lamp, etc., or it may operate with the door locking.

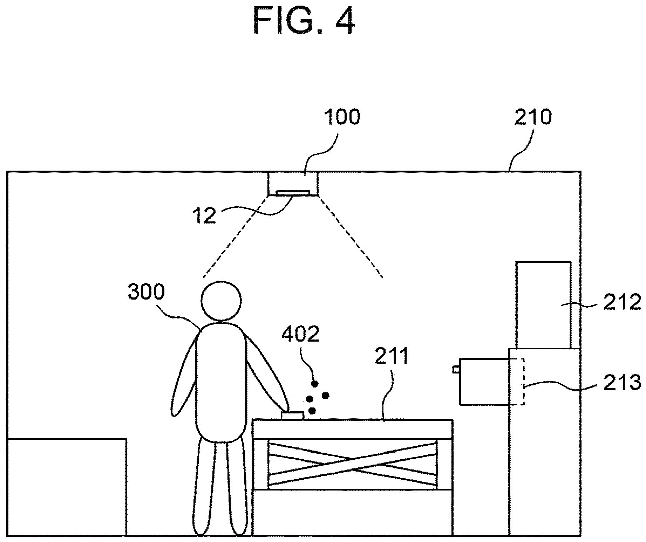

As shown in FIG. 4, the UV irradiation apparatus 100 continues the UV irradiation after the worker 300 has entered the treatment chamber 210. The UV irradiance at this time may be the same as that in the unmanned condition, or alternatively, it may be lower than the UV irradiance in the unmanned condition, taking into account the situation that the treatment chamber 210 is manned.

When the worker 300 is wearing protective clothing and the protective clothing is made of a material that blocks (or inhibits) the transmission of the UV light, the UV irradiance may be relatively high because the worker 300 is shielded by a shielding material that blocks the transmission of the UV light.

As shown in FIG. 4, the worker 300 wipes down the surfaces in the treatment chamber 210 while the UV irradiation is being performed. At this time, the worker 300 wipes down areas that are not sufficiently irradiated with the UV light, such as the interior of the drawer 213 and the surfaces of complex equipment. In addition, the worker 300 wipes off objects to be cleaned 401, such as large droplets, blood, and the like, which cannot be fully decontaminated by UV irradiation alone.

In this way, by performing the UV irradiation in an unmanned state in advance, the worker 300 may perform the wiping operation selectively on areas that cannot be covered by the UV irradiation (i.e., areas that have been insufficiently inactivated) rather than all over the surface in the treatment chamber 210.

To wipe off the object to be cleaned 401 shown in FIG. 3, when a worker 300 sprays a sterilizing solution to the object to be cleaned 401 or wipes down a surface on which the object to be cleaned 401 is adhered, droplets 402 will fly up from the object to be cleaned 401 into the space, as shown in FIG. 4. When the above sterilizing solution is a volatile sterilizing solution such as alcohol, the droplets 402 will fly up as well when the sterilizing solution volatilizes. Assuming that the droplets contain infectious bacteria, or the like, the worker 300 is likely to be infected once the worker 300 inhales the droplets.

According to the present embodiment, by performing the UV irradiation simultaneously with the wiping operation, it makes it possible to irradiate the droplets 402 that are scattered from the wiping area during the wiping operation with the UV light. Furthermore, by irradiating the space between the wiping area and the worker 300 with the UV light, it makes it possible to irradiate the droplets 402 immediately after the droplets 402 fly up from the wiping area with the UV light. As a result, it makes it possible to inactivate the viruses, or the like, contained in the droplets 402 in the space before the worker 300 inhales the droplets 402 that spread out during wiping operation, thereby preventing secondary infection to the worker 300.

Subsequently, the worker 300 exits the treatment chamber 210 when the wiping operation is completed. At this time, the control unit 16 of the UV irradiation apparatus 100 turns off the excimer lamp 20 and terminates the UV irradiation at the timing when the worker 300 exits the treatment chamber 210. In this case, the control unit 16 may turn off the excimer lamp 20 based on a lighting-off instruction (e.g., by operating a lighting switch) from the worker 300 exiting the treatment chamber 210, or alternatively, the control unit 16 may turn off the excimer lamp 20 at the timing when the worker 300 is detected to have left the treatment chamber 210 by various sensors.

It should be noted that the timing for terminating the UV irradiation is not limited to when the worker 300 exits the treatment chamber 210. For example, the UV irradiation may be continued after the worker 300 exits the treatment chamber 210, and then terminated when it may be determined that sterilization and inactivation has been sufficiently achieved in the treatment chamber 210.
According to the ACGIH (American Conference of Governmental Industrial Hygienists) and JIS Z 8812 (Measurement Method of harmful ultraviolet radiation), the irradiation amount of UV light to the human body per day (i.e., 8 hours) is specified as the allowable limit value (TLV: Threshold Limit Value) for each wavelength. Although those TLVs may be revised in the future, it is appropriate to ensure that the irradiation amount of UV light (i.e., integrated luminous intensity) does not exceed the specified TLVs. Therefore, taking the above mentioned TLVs into consideration, for example, UV irradiation may be terminated when a predetermined time has elapsed after the worker 300 enters the treatment chamber 210.

During the UV irradiation in the manned state, the control unit 16 may control the amount of UV radiation emitted from the excimer lamp 20 based on signals from the human-detecting sensor 31 and the proximity sensor 32. For example, when the control unit 16 receives a detection signal from the human-detecting sensor 31 indicating that a human is detected within the irradiation area and a detection signal from the proximity sensor 32 indicating that an object within the detection range is detected, the control unit 16 determines that a human is present within the detection range and may control the amount of UV radiation from the excimer lamp 20 to be reduced.

The control to reduce the amount of UV radiation emitted from the excimer lamp 20 may include the control to turn off the excimer lamp 20, the control to reduce the irradiance (i.e., radiant illuminance) of the UV light emitted from the excimer lamp 20, and the control to reduce the lighting duty ratio of the excimer lamp 20. Here, the lighting duty ratio is the ratio of the lighting time with respect to the sum of the lighting time during which the UV light source is being turned on and the lighting-off time during which the UV light source is being turned off.

Here, in the control to reduce the irradiance of the UV light, the reduction amount in irradiance of UV light may be a fixed amount, a fixed percentage with respect to the irradiance of the normal lighting, or alternatively, an amount depending on the distance to the object detected by the proximity sensor 32, for example, the closer the distance, the greater the reduction amount. In the control to reduce the lighting duty ratio, the lighting duty ratio after reduction in irradiance of UV light may be a fixed value, or alternatively, it may be a value depending on the distance to the object detected by the proximity sensor 32, for example, the closer the distance, the smaller the lighting duty ratio.

It should be noted that the pyroelectric infrared sensor, which is a common human-detecting sensor, cannot detect a human who completely remains still. Therefore, assuming that the human-detecting sensor 31 and the proximity sensor 32 are used together, when a human remains still in an area within the predetermined separation distance from the light emitting surface 12, that human cannot be detected by the human-detecting sensor 31, and the control of reducing the irradiance of UV light would not be initiated even when an object is detected by the proximity sensor 32. However, that is not the case because the worker 300 is wiping the interior of the vehicle and thus the worker 300 is unlikely to remain still.

As described above, according to the present embodiment, the UV light is irradiated onto the interior of the ambulance 200, which is an enclosed space where patients are transported and treated, from the UV irradiation apparatus 100 installed in the ambulance 200 so as to inactivate harmful microorganisms and/or viruses existing in the ambulance 200. Here, the UV irradiation apparatus 100 includes the light source unit and the control unit 16 that controls the lighting of the light source unit. The light source unit includes the excimer lamp 20 that emits light including UV light having the wavelength range between 190 nm and 235 nm, and the housing 11 with the light emitting surface 12 that emits light emitted from the excimer lamp 20.

As such, using the UV light having the wavelength range between 190 nm and 235 nm, which have little adverse effects on cells of humans and animals, it makes it possible to sterilize and inactivate the interior of the ambulance 200 by irradiating with UV light, even when a human is present in the ambulance 200.

According to the present embodiment, the present method simultaneously performs the wiping process (i.e., first step), in which a worker 300 wipes down the surfaces inside the ambulance 200 after a patient has been transported with a sterilizing solution, and the UV irradiation process (i.e., second step), in which the UV light having the wavelength range between 190 nm and 235 nm is irradiated into the interior the ambulance 200 as the UV light that inactivates harmful microorganisms and/or viruses. Here, in the UV irradiation process, which is performed simultaneously with the wiping process, the above UV light is irradiated onto the droplets that scatter from the wiping area during the wiping operation.

As such, since the wiping operation using the sterilizing solution and the sterilization and inactivation processing using the UV light are performed simultaneously, it makes it possible to appropriately perform the sterilization operation inside the ambulance 200 in a short period of time after a patient being transported and before the next dispatch. As a result, it makes it possible to operationalize ambulances 200 more efficiently.

Furthermore, by irradiating the droplets scattered from the wiping area during the wiping operation with the UV light, even when the droplets contain harmful microorganisms and/or viruses, it makes it possible to inactivate the above microorganisms and/or viruses by the UV light before the droplets are inhaled by the worker 300. As a result, it makes it possible to appropriately prevent secondary infection to the worker 300.

Yet furthermore, by installing the UV irradiation apparatus 100 at a position where the UV light can be irradiated in the space between the wiping area and the worker 300, it makes it possible to irradiate the droplets with the UV light immediately after the droplets have scattered from the wiping area during the wiping operation. Therefore, it makes it possible to inactivate microorganisms and/or viruses contained in the droplets by UV irradiation more appropriately before the worker 300 inhales the droplets.

Yet furthermore, by performing the UV irradiation simultaneously with the wiping operation, it makes it possible for the worker 300 to perform the wiping operation selectively on areas where inactivation by the UV irradiation is determined to be insufficient, such as areas where large droplets still exist that cannot be inactivated by a certain amount of UV irradiation. Therefore, it makes it possible to reduce the workload of the worker 300.

Conventionally, a certain technology has been known to sterilize by spraying ozone in a space, however, ozone has a strong oxidizing effect and thus deteriorates resins and rubbers. In this respect, an ambulance is supposed to contain resin-made and rubber-made components, such as connecting wires for measuring instruments, medical bands, gloves, and the like. For this reason, in the case of ozone sterilization, those components inside the ambulance may be deteriorated by the oxidizing action of ozone. According to the present embodiment, it makes it possible to suppress the above deterioration by using the sterilizing solution and the UV light having the wavelength range that does not produce ozone.

According to the present embodiment, prior to the wiping process and the UV irradiation process described above, the present method may perform another UV irradiation process (i.e., third step) in which the UV light is irradiated onto the interior of the ambulance 200 in an unmanned state with no human present in the ambulance 200 after a patient has been transported.

In this case, the worker 300 may enter the ambulance 200, which has been decontaminated to some extent by the UV irradiation, to perform the wiping operation. As a result, it makes it possible to reduce the risk of infection to the worker 300, and also reduce the psychological anxiety of the worker 300.

Yet furthermore, by exposing the interior of the drawer 213 at this time (i.e., fourth step), it makes it possible to irradiate the area including the exposed interior of the drawer 213 with the UV light. In this case, it makes it possible to eliminate or at least simplify the wiping operation of the interior of the drawer 213.

As described above, according to the inactivation method of the present embodiment, it makes it possible to inactivate microorganisms and/or viruses existing in the space where patients are transported and/or treated and sterilize the space in a short period of time and safely to humans.

### Modification to Embodiments

Although the above embodiment describes a certain case in which the space to be inactivated is an enclosed space where patients are transported and treated, the target space to be inactivated may suffice as long as the target space may be an enclosed space where patients are transported and/or treated.

Furthermore, although the above embodiment describes a certain case in which the enclosed space to be inactivated is a space inside an ambulance, it is not limited thereto, and the target space to be inactivated may be a space in a transport vehicle such as a doctor car. Also, the above enclosed space is not limited to the space inside a vehicle, but may also be a space inside a medical facility such as an operating room, intensive care unit, or hospital room, for example.

Yet furthermore, although the above embodiment describes a certain case in which the human-detecting sensor 31 and the proximity sensor 32 are arranged in the housing 11 of the UV irradiation apparatus 100, alternatively, the human-detecting sensor and the proximity sensor may be separate from the UV irradiation apparatus 100. It may suffice to arrange the human-detecting sensor 31 at a location where the human-detecting sensor 31 is able to detect a human present within the area (i.e., irradiation area) to be irradiated by the UV light emitted from the light emitting surface 12. Likewise, it may suffice to arrange the proximity sensor 32 at a location where the proximity sensor 32 is able to detect an object present within the detection range, which is an area within a predetermined proximity distance from the light emitting surface 12. In this case, the control unit 16 of the UV irradiation apparatus 100 receives detection signals transmitted from the external human-detecting sensor and the proximity sensor, and controls the reduction of the UV radiation based on the received detection signals.

Yet furthermore, although the above embodiment describes a certain case in which the excimer lamp 20, which is an UV light source, has a configuration in which a pair of electrodes 22 and 23 are arranged on one side of the discharge vessel 21, as shown in FIG. 2, the configuration of the excimer lamp is not limited to the above.

For example, the excimer lamp may be configured with a pair of annular electrodes (i.e., first and second electrodes) arranged at both ends of a long discharge vessel, respectively. The excimer lamp may also be configured with an inner electrode (i.e., first electrode) inside the long discharge vessel and a mesh (i.e., net-shaped) or linear outer electrode (i.e., second electrode) on the outer wall surface of the discharge vessel. As yet another example, a so-called "flat tube structure" may be employed, which has the first and second electrodes on the two facing outer surfaces of the flat discharge vessel, respectively. Yet alternatively, a so-called "double-tube structure" may also be employed, which has a cylindrical outer tube and a cylindrical inner tube. In this case, the excimer lamp may be configured to include a mesh-shaped first electrode (i.e., outer electrode) and a membrane-shaped second electrode (i.e., inner electrode) on the outer surface of the outer tube and the inner surface of the inner tube, respectively.

Yet furthermore, although the above embodiment describes a certain case in which excimer lamps are used as the UV light source, alternatively, LEDs may also be used as the UV light source.

For example, aluminum gallium nitride (AlGaN)-based LEDs, aluminum nitride (AIN)-based LEDs, magnesium zinc oxide (MgZnO)-based LEDs, and the like, may be employed as the LED light source.

Here, for AIGaN-based LEDs, it is preferable to adjust the composition of Al such that the center wavelength is within the range between 190 nm and 235 nm. AIN-based LEDs emit UV light with a peak wavelength of 210 nm. MgZnO-based LEDs may also emit UV light with a center wavelength of 222 nm by adjusting the composition of Mg.

As described above, according to the inactivation method of the present invention, by using the UV light having the wavelength between 190 nm to 235 nm, it makes it possible to provide the inherent germicidal and virus inactivation capabilities of UV light without incurring the adverse effects of UV irradiation on human bodies. In particular, unlike conventional UV light sources, it makes it possible to perform the inactivation processing even in spaces where a human is present more effectively.
This addresses Goal 3 of the United Nations-driven Sustainable Development Goals (SDGs): "Ensure healthy lives and promote well-being for all people of all ages," and make a significant contribution to Target 3.3: "By 2030, end the epidemics of AIDS, tuberculosis, malaria and neglected tropical diseases and combat hepatitis, water-borne diseases and other communicable diseases."

### REFERENCE SIGNS LIST

11: Housing; 12: Light Emitting Surface; 15: Power Supply Unit; 17: Control Unit; 20: UV Light Source; LEDs; 31: Human-Detecting Sensor; 32: Proximity Sensor; 100: UV Irradiation Apparatus; 200: Transport Vehicle (Ambulance); 201: Ceiling; 202: Handrail; 203: Wall; 210: Treatment Chamber; 211: Stretcher; 212: Equipment; 213: Drawer; 300: Worker; 401: Object to be Cleaned; 402: Droplets

## Claims

1. An inactivation method of inactivating harmful microorganisms and/or viruses existing in an enclosed space where a patient is transported and/or treated, comprising:
a first step of performing a wiping operation that wipes down a surface in the enclosed space with a sterilizing solution after transporting and/or treating the patient;
a second step of irradiating, simultaneously with the first step, droplets that scatter from a wiping area of the surface during the wiping operation in the first step with ultraviolet light in a wavelength range between 190 nm and 235 nm as ultraviolet light having a wavelength that inactivates harmful microorganisms and/or viruses.

2. The inactivation method according to Claim 1, further comprising:
a third step of irradiating, prior to the first step and the second step, the enclosed space with the ultraviolet light in an unmanned state where no human is present in the enclosed space after transporting and/or treating the patient.

3. The inactivation method according to Claim 2, wherein
the enclosed space is a space where a shelf storing equipment used for a treatment for the patient is arranged,
the inactivation method further comprises a fourth step of exposing an interior of the shelf after transporting and/or treating the patient, and
the third step irradiates an area including the exposed interior of the shelf with the ultraviolet light.

4. The inactivation method according to Claim 1, wherein
the sterilizing solution is a volatile sterilizing solution.

5. The inactivation method according to Claim 1, wherein
the second step irradiates the droplets with the ultraviolet light by irradiating a space between the wiping area and a worker performing the wiping operation.

6. The inactivation method according to Claim 1, wherein
the first step performs the wiping operation selectively on an area of the surface in the enclosed space where inactivation of the microorganisms and/or viruses by irradiation with the ultraviolet light is determined to be insufficient.

7. The inactivation method according to any one of Claims 1 to 6, wherein
the enclosed space is a space inside an ambulance.
